(19) Europäisches Patentamt
European Patent Office
Office européen des brevets

(11) EP 1 080 694 A1

(12) **EUROPEAN PATENT APPLICATION**

(43) Date of publication:
**07.03.2001 Bulletin 2001/10**

(51) Int. Cl.[7]: **A61B 18/12**

(21) Application number: **00304252.0**

(22) Date of filing: **19.05.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **11.06.1999 GB 9913652**

(71) Applicant: **GYRUS MEDICAL LIMITED**
**Cardiff CF3 0LT (GB)**

(72) Inventors:
- **Hancock, Christopher Paul**
  **Cardiff CF2 3EX (GB)**
- **Amoah, Francis Egyin**
  **Whitchurch, Cardiff CF14 1TE (GB)**
- **Penny, Keith**
  **Monmouth NP25 5AD (GB)**
- **Goble, Colin Charles Owen**
  **Penarth, South Glamorgan CF64 1AT (GB)**

(74) Representative:
**Blatchford, William Michael et al**
**Withers & Rogers**
**Goldings House,**
**2 Hays Lane**
**London SE1 2HW (GB)**

(54) **An electrosurgical generator.**

(57) An electrosurgical generator has sensing circuitry for sensing changes in reflected power fed back from a radio-frequency output of the generator, and a control arrangement coupled to the sensing circuitry for processing a reflected power monitoring signal. The control arrangement acts to reduce or stop the application of radio frequency power to the generator output in response to a predetermined condition of the monitoring signal indicative of a change in reflected power due to a change in the state of the tissue being treated. The generator has upper and lower frequency parts operable above and below 300MHz, both parts being connected to the generator output, and the control arrangement reduces or cuts off the applied radio frequency power when the load impedance sensed at the operating frequency of the lower frequency part changes.

*FIG. 1A*

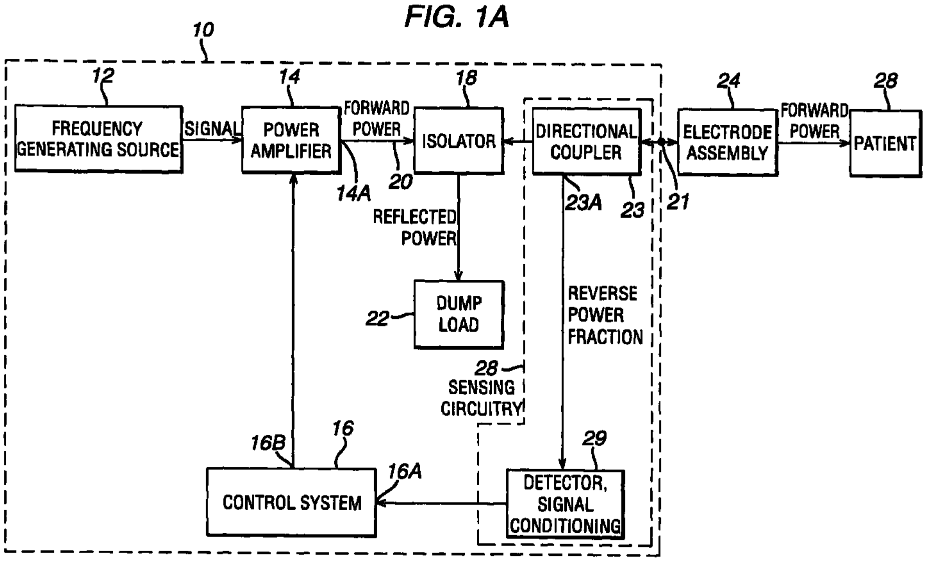


EP 1 080 694 A1

## Description

This invention relates to an electrosurgical generator the output power of which is automatically adjustable.

In electrosurgery it is desirable to be able automatically to adjust power delivery to the tissue being treated, particularly in circumstances of a required tissue change having been achieved. When living tissue or neighbouring material is affected by the application of radio frequency (r.f.) energy at electrosurgical levels, changes occur in its electrical characteristics, especially electrical conductivity and dielectric constant. Consequently, the electrical load impedance presented to an electrosurgical instrument by the tissue or other material changes as a function of the state of the tissue or the other material.

According to a first aspect of this invention, an electrosurgical generator comprises a radio frequency power source coupled to an output of the generator, which output is for connection to an electrosurgical electrode assembly, sensing circuitry operable to sense changes in reflected power fed back from the output, and a control arrangement having an input coupled to the sensing circuitry to process a monitoring signal from the sensing circuitry, and a power control output coupled to the power source for adjusting the power output of the source, wherein the control arrangement is adapted to reduce or stop the application of radio frequency power from the source to the generator output in response to a predetermined condition of the monitoring signal indicative of a change in reflected power due to a change in the state of the tissue being treated.

In a preferred generator, the r.f. power source is arranged to operate at 300MHz or upwards, and a three port impedance isolating device is coupled with a forward power path between the r.f. power source and the generator output, and a reflected power path between the generator output and a resistive dump load. The sensing circuitry may include a directional coupler associated with a reflected power path section of an output line between the r.f. power source and the generator output or between the isolating device and the dump load. In its preferred form, the sensing circuitry is arranged to provide a voltage or current signal representative of power reflected back from the generator output, this signal being fed to a detector and a signal conditioning stage which together provide the monitoring signal. The impedance isolating device may comprise a circulator or hybrid coupler.

With such a generator, it is possible to terminate tissue changes once a required tissue state has been achieved through sufficient energy transfer and no more tissue changes are required (for example when welding two tissue structures together). Undesirable tissue effects may be avoided or minimised as, for instance, in the delivery of r.f. power as an efficient means of coagulation to staunch bleeding. It is desirable, in this case, to cease application of energy before the coagulant adheres to an electrode of an electrode assembly, in order to prevent tearing of the tissue when the electrode is moved. The generator is also useful when two stage treatment is required as in the case of carrying out preparatory coagulation of tissue using UHF energy prior to tissue cutting and/or vaporisation using low frequency energy. In this case, the sensing circuitry and the control arrangement may be operated together to sense completion of the preparatory treatment in order to alter the r.f. power source output from being one in which energy is principally supplied in a first frequency range to one in which energy is principally supplied in a second, different frequency range.

During electrosurgery, repetitive changes of tissue or associated material states may be observed. For instance, liquid within the tissue being treated or liquid in the region of the tissue being treated may boil during treatment, or material adjacent the electrode may move in an oscillatory fashion, both effects giving rise to modulation of the reflected power signal due to repetitive changes in load impedance. Such modulation may be detected by a demodulator forming part of the sensing circuitry, the control arrangement and the signal conditioning stage being adapted to cause a reduction in the output power of the power source when the modulation, as represented by the detection signal, exceeds a predetermined threshold level, or when the detection signal indicates that modulation is reducing or has ceased after a period during which it is present.

As an alternative, the rate of change of reflected power or modulation of the reflected power may be sensed, according to the tissue state to be monitored. The reflected power signal may be applied to a dual-channel detection and signal conditioning arrangement in which one channel includes, for instance, averaging means such that a comparator having inputs connected to the outputs of the respective channels produces a sensor output signal representative of short term changes in the relevant property of the reflected power. This allows relatively slow effects such as changes in impedance and reflected power due to movement of the treatment electrode to be cancelled out.

Taking a general view, then, the invention makes use of high frequency reflected power changes occurring as a result of differences in tissue impedance and feed structure. A detector forming part of the generator and connected to the generator output to measure reflected power may produce voltage or current signals containing amplitude and/or phase information from which a determination as to tissue state may be made automatically. As the electrical characteristics of the tissue change, the amplitude and phase of the reflected signal varies. Some tissue effects cause rapid fluctuations in load impedance presented to the electrode which result in amplitude, phase, or frequency modulation. The presence of modulation and the characteristics of such modulation may indicate tissue changes or other processes which may be exploited to control power delivery in accordance with the invention as the tissue changes state to achieve optimal power delivery and to control the rate of change in the tissue.

According to a second aspect of the invention, an electrosurgical generator comprises a radio frequency (r.f.) power source having an upper frequency part operable above 300 MHz and a lower frequency part operable below 300 MHz, a generator output for connection to an electrosurgical electrode assembly, both parts of the power source being coupled to the output, sensing circuitry including a load impedance sensor for sensing load impedance at a frequency or frequencies of operation of the lower frequency part of the source, and a control arrangement having an input coupled to the sensing circuitry to process a monitoring signal from the sensing circuitry, and a power control output coupled to the power source for adjusting the power output of the source, wherein the control arrangement is adapted to reduce or stop the application of r.f. power from the upper frequency part of the source to the generator output in response to a predetermined condition of the monitoring signal indicative of a change in load impedance sensed at the said operation frequency or frequencies of the lower frequency part of the source. The load impedance sensor may comprise voltage and current measuring circuitry coupled to an output line between the lower frequency part of the source and the generator output. Alternatively, in the case of the frequency of operation of the lower frequency part of the source being dependent on load impedance, the load impedance sensor senses the frequency of the lower frequency part.

It follows that the delivery of power at frequencies above 300 MHz may be controlled not only by monitoring reflected power above 300 MHz, but also by monitoring the load impedance presented to the generator output at frequencies below 300 MHz, depending on the condition of the tissue being treated or the mode of surgical treatment (e.g. coagulation or tissue cutting and/or vaporisation). When load impedance is being sensed, the application of power from the upper frequency part of the source may be reduced or stopped by the control arrangement when the sensed load impedance presented to the generator output at the frequency or frequencies of operation of the lower frequency part exceeds a predetermined threshold. It is not necessary for the lower frequency part to deliver power at a level sufficient to achieve an electrosurgical effect. Indeed, it may produce a signal of a level merely sufficient for the sensing circuitry to be able to produce a monitoring signal indicative of load impedance.

The invention will now be described by way of example with reference to the drawings in which-

Figure 1A, 1B and 1C are block diagrams of three electrosurgical systems each including an electrosurgical generator in accordance with the invention;

Figures 2A, 2B, and 2C are block diagrams of alternative detector and signal conditioning stages for the generators of Figure 1A, 1B and 1C;

Figure 3 is a block diagram of a fourth electrosurgical system in accordance with the invention; and

Figure 4 is a block and circuit diagram showing part of the system of Figure 3 in greater detail.

Referring to Figure 1A, a generator 10 in accordance with the invention has a radio frequency source comprising a frequency generating source 12 and a power amplifier 14. Source 12 may be in the form of one or more frequency synthesisers, the frequencies of which are controlled by a control system 16.

The preferred generator supplies electrosurgical power at UHF via an impedance isolator 18 such as a circulator which couples a forward power path as an output line 20 between the power amplifier output 14A and a generator output terminal 21. In a reverse power path, the impedance isolator also couples the generator output terminal 21 to a dump load 22 via a directional coupler 23 with the purpose of generating a reverse power fraction signal at a reverse power output 23A. In Figure 1A, the output terminal 21 is shown connected to an electrode assembly 24 having a treatment electrode which, during use is applied to the patient 28 and, specifically, to the tissue to be treated.

The impedance isolator 18 and the dump load 22 cause a nominal 50 ohm impedance to be presented to the power amplifier output 14A. They may be omitted if the power amplifier output 14A is sufficiently rated.

The directional coupler 23 is connected in series between the radio frequency source 12, 14 and the electrode assembly 24, and forms part of sensing circuitry 28. The reverse power output 23A of the coupler, providing a reverse power fraction signal representative of reflected power, is coupled to a detection and signal conditioning stage 29 for processing the reverse power fraction signal.

Unless a perfect conjugate impedance match exists between the generator 10 and the electrical load represented by the electrode assembly 24 and its surroundings (including patient 26), some of the power fed from the output terminal 26 of the generator to the electrode assembly is returned as reflected power. This is measured by a receiver or detector in the detection and signal conditioning stage 29 as a voltage which provides a reflected power output signal indicative of the magnitude of the reflected power.

Further processing of this signal is performed the signal conditioning stage 29 to process, for instance, modulation of the reflected power, giving rise to a monitoring signal which is fed to a monitoring input 16A of the control system 16. The control system 16 makes use of the sensor output signal to determine when tissue changes are taking place and as a result, to reduce or terminate the power output of the generator by means of a control output 16B coupled to the power amplifier 14.

Referring to Figure 1B, an alternative arrangement for measuring reflected power has a directional coupler 23' positioned in the reverse power path between the impedance isolator 18 and the dump load 22. The advantage of this orientation is that the power rating requirement of the directional coupler is lower, whereas the previous embodiment gives the opportunity to use a bi-directional coupler and combine reverse power measurement and forward power measurement for other control purposes.

Referring to Figure 1C, in a third embodiment the reverse power feedback fraction signal is taken from the output of a resistive high power attenuator 30 which additionally serves as a dump resistor for reverse power from the generator output terminal 21.

Three alternative exemplary configurations of the detection and signal conditioning stage 29 are now described with reference to Figures 2A, 2B, and 2C which disclose a detector 40 for detecting modulation in the reflected power present at the output 21 of the generator.

The detector 40 is an amplitude modulation (AM) detector having an output 40A. The output 40 carries the detected amplitude modulation. Referring to Figure 2A, the control system includes a comparator 44 having one input connected directly to the AM output 40A of the detector 40 and the other input connected to receive a time averaged representation of the signal fed from the AM output 40A of the detector 40. Averaging is performed by a time averaging module 46. The time constant $T_1$ of the averaging module 46 is arranged such that the signal fed to the second input of the comparator constitutes a reference level which varies according to relatively slow changes in the amplitude of the reflected power signal due to, for instance, changes in orientation of the electrode assembly relative to the tissue being treated. In this way, the output signal obtained from the comparator 44 is made representative predominantly of rapid changes in the reflected power signal or its depth of modulation due to rapid changes in tissue state such as boiling of liquids in the tissue or adjacent the tissue.

The comparator output signal is applied to a power controller part of the control system for controlling the output power level of the power amplifier 14 of the radio frequency source (see Figures 1A, 1B, 1C) via the power control connection 16B between the control system 16 and the power amplifier 14. The power controller is configurable to adjust the power delivered by the power amplifier 14 in response to AM representative of liquid boiling adjacent the treatment electrode or when boiling has been detected and then ceases. Depending on the tissue effect to be achieved, the power controller of the control system 16 may be configured to reduce the output power level of the amplifier 14 when amplitude modulation is detected (for example, when the depth of modulation exceeds a predetermined threshold), the reduction taking place progressively until the comparator output signal changes state, indicating a reduction in boiling. Power may then be increased in comparatively small steps until the comparator output level again changes state or until an initially set output power level of the power amplifier 14 has been reached.

Alternatively, the power output from the power amplifier 14 may be reduced when a cessation of boiling-indicative AM occurs.

These techniques may be used to prevent sticking of tissue and/or coagulant to the treatment electrode of the electrode assembly 24.

In a second version of the detection and signal conditioning stage, as shown in Figure 2B, the amplitude modulation from the first output 40A of the detector 40 may be differentiated to obtain a slope signal for feeding to the first input at the comparator 44, differentiation being performed by a differentiator module 50. This yields a comparator output signal which is responsive to extremely rapid changes in reflected power level, represented by amplitude modulation spikes, indicative of vigorous boiling at the electrode.

In a further alternative, illustrated in Figure 2C, similar discrimination between amplitude modulation indicative of tissue changes and longer term changes due to orientation and proximity of the electrode with respect to the tissue is achieved by coupling the output 40A of the detector 40 respectively to first and second time averaging circuits 54, 56 having, respectively, short and long time constants $T_1$ and $T_2$, the outputs of these time averaging circuitry being connected respectively to the first and second inputs of the comparator 44. The time constant $T_2$ is in the region of ten times greater than the time constant $T_1$.

It will be understood by those skilled in the art that many functions described above can be carried out as software steps in a microcontroller, including processing of the detected modulation signal, time averaging, and comparison. The invention as claimed includes both hardware and software variants within its scope.

In the embodiments described above, the application of electrosurgical power at UHF is controlled by monitoring the reflected or reverse power in the output circuit of the generator. Whilst the reflective power signal from the generator output may indicate the onset of tissue desiccation, under some circumstances it is advantageous to continue delivering power at UHF or higher frequencies beyond the tissue moisture vaporisation stage. Once such moisture has been largely dispersed, it is advantageous to monitor tissue state by sensing the load impedance presented to the generator output. This is best performed in the HF/VHF region between 100 kHz and 40 MHz, and preferably between 300 kHz and 5 MHz.

Referring to Figure 3, an electrosurgical system capable of monitoring load impedance in this way has a generator with an upper frequency source part 62 for delivering a signal at UHF or EHF frequencies, typically 2.45 GHz, and a lower frequency source part 64 for delivering a signal at HF/VHF, preferably in the region of 1 MHz. As in the embodiments described above with reference to Figures 1A to 1C, the UHF/EHF source drives a UHF/EHF power amplifier 14 which produces a UHF/EHF forward power signal at its output 14A for application to an impedance isolator 18. Isolator 18 is coupled to the generator output terminal 21 in this case via a combiner circuit 66. Power reflected from the load at the operating frequency of the UHF/EHF source is directed by the impedance isolator 18 to a dump load 22.

The lower frequency part 64 of the power source drives an HF/VHF power amplifier 68 which feeds a signal at the lower frequency, i.e. the frequency of operation of the source part 64, through a voltage and current sensing stage 70 and a reactive compensation stage 72 to a second input of the combiner 66.

Accordingly, the generator is capable of supplying electrosurgical power at the output terminal 21 at both upper and lower frequencies of 2.45 GHz and 1 MHz respectively. As before, the level of the electrosurgical signal delivered to the generator output 21 by the power amplifier 14 is controlled by a control system 16 via its control output 16B. The control system has a load impedance monitoring input 16D for receiving a monitoring signal from the voltage and current sensing stage 70, which signal is representative of the load impedance presented to the generator output 21 measured at the frequency of the lower frequency part 64 of the source. The action of the combiner 66 and the reactive compensation stage 72 is such that the monitoring signal is largely responsive to load impedance changes at the lower frequency only, i.e. within a 100kHz to 40 MHz band, or 300 kHz to 5 MHz band.

In operation, the voltage and current sensing stage 70 senses amplitude and phase information relating to the voltage and current supplied at the lower frequency to the generator output terminal 21 to derive a monitoring signal which can be used as a measure of load impedance at the lower frequency. In this way, it is possible to regulate, reduce, or cut off the delivery of electrosurgical power from the power amplifier 14 when, for instance, the sensed lower frequency load impedance reaches a predetermined level indicative of a required tissue condition. This may be used particularly to confine treatment to coagulation or desiccation of tissue when in a coagulation or desiccation mode; in other words to prevent tissue vaporisation or ablation when not required. Equally, an impedance or resistance threshold may be used to determine the completion of a preparatory coagulation treatment prior to tissue vaporisation, the control system being configured to apply a tissue vaporisation signal to the generator output terminal 21 when the predetermined threshold has been reached. This may be performed by switching from a condition in which electrosurgical power is supplied predominantly at the upper frequency to a condition in which electrosurgical power is supplied predominantly at the lower frequency.

In some circumstances, it is useful to be able to control the frequency of operation of the lower frequency part 64 of the radio frequency source. This may be achieved via a frequency control output 16E from the control system, coupled to a frequency control input of the source part 64.

The manner in which impedance sensing is performed will now be described in more detail with reference to Figure 4. Here, the lower frequency source part 64 and the power amplifier 68 are seen as a simple HF/VHF voltage source 64, 68 which feeds a signal, typically at 1 MHz, via a shunt-connected parallel-resonant circuit acting as the reactive compensation stage 72, the parallel-resonant circuit comprising an inductor 72L and a capacitor 72C. The load at the lower frequency due to the electrode assembly 74 attached to the generator output terminal 21, and due to the tissue of the patient 28 being treated, are represented respectively by a parallel capacitor 74C, a tissue resistance 76 and a series coupling capacitance 78. Lower frequency voltage and current signals are picked up respectively by a voltage sensing transformer 80 connected in parallel, and a current sensing transformer 82 connected in series in one of the output lines of the source 64, 68. These transformers 80, 82 have secondary windings coupled to respective input terminal pairs of a signal conditioning stage 84 which yields three output signals at its outputs 84A, 84B, 84C representing voltage amplitude, current amplitude, and the phase difference ($\phi$) between the voltage and current signals.

The control system 16 receives these signals at its low frequency sensing input 16D and computes the load impedance (and hence the tissue impedance) by combining the voltage and current amplitudes (VI), and the phase difference $\phi$ according to the expression $V/(I \cos \phi)$. This computation is represented in Figure 4 as a computation stage 16P having an output terminal 16PA which drives a comparator module 16Q which, in turn, drives an output amplifier 16R. Comparitor 16Q acts in the manner of a differential amplifier having a second input which receives a signal representative of a tissue impedance or resistance threshold as a reference signal so that the output amplifier 16R produces an output proportional to the difference between the sensed impedance and the impedance reference, the amplifier 16R having a second input for receiving a reference signal representing an upper power limit so that when the sensed impedance reaches the predetermined threshold, the power delivered from the power amplifier 14 (see Figure 3) is folded back from the upper limit, in other words reduced from the upper limit.

Alternative control actions may be implemented by, for instance, causing complete cessation of UHF/EHF power delivery upon reaching a given tissue impedance.

It will be appreciated that impedance sensing at the lower frequency may be performed without generating a lower frequency signal (in source 64, 68) of a level sufficient to cause electrosurgical effects. Consequently, when treatment at the lower frequency is not required, a lower frequency signal is supplied at a level merely sufficient to carry out impedance sensing, the transformers 80, 82 and the signal conditioning stage 84 being arranged so as to be appropriately sensitive. Delivery of an r.f. signal at the lower frequency at a level sufficient merely for measurement rather than tissue effect obviates a need to prevent earth leakage.

Although Figure 3 does not indicate control of the UHF/EHF power output in response to reflected power as described above with reference to Figures 1A to 1C, it will be understood that the generator of Figure 3 has the facility to perform both sensing functions, the reflected power sensing stages being omitted merely for clarity. However, a system or generator making use of lower frequency impedance sensing alone falls within the scope of the invention in accordance with at least one if its aspects.

The way in which tissue impedance or resistance varies with time during tissue coagulation and desiccation is illustrated in US Patent No. 5,423,810, the contents of which are incorporated herein by reference.

It is also possible to configure the lower frequency part of the generator as a variable frequency power oscillator which is self-tuning in the sense that its output frequency is dependent upon load impedance, as described in the Applicant's British Patent Application No. GB2214430A. In this case, the oscillator frequency may be used as a monitoring signal representative of impedance, the generator including an impedance sensing stage employing a frequency counter or alternative frequency-responsive circuit coupled to the output of the source 64, 68.

As with the embodiments described above with references to Figure 1A to 1C, it will be appreciated that the control system 16 and, depending on the frequencies involved, part of the signal conditioning stage 84 may be implemented as software steps in a microcontroller.

## Claims

**1.** An electrosurgical generator comprising a radio frequency (r.f.) power source coupled to an output of the generator, which output is for connection to an electrosurgical electrode assembly, sensing circuitry operable to sense changes in reflected power fed back from the output, and a control arrangement having an input coupled to the sensing circuitry to process a monitoring signal from the sensing circuitry, and a power control output coupled to the power source for adjusting the power output of the source, wherein the control arrangement is adapted to reduce or stop the application of r.f. power from the source to the generator output in response to a predetermined condition of the monitoring signal indicative of a change in reflected power due to a change in the state of the tissue being treated.

**2.** A generator according to claim 1, wherein the r.f. power source is arranged to operate at 300MHz or upwards and the sensing circuitry includes a directional coupler associated with an output line between the r.f. power source and the generator output and which has a reflected power output for providing a voltage or current signal representative of power reflected back from the generator output.

**3.** A generator according to claim 1, wherein the r.f. power source is arranged to operate at 300MHz or upwards, and the generator includes an impedance isolation device coupled in an output line between the source and the generator output, and wherein the sensing circuit has an input coupled to a reflected power output of the isolation device.

**4.** A generator according to claim 1 or claim 2, wherein the sensing circuitry is arranged to generate the said monitoring signal as a function of the rate of change in the reflected power.

**5.** A generator according to any preceding claim, wherein the sensing circuitry is associated with an output line coupling the power source to the generator output and is arranged to generate a reflected power signal which is a voltage or current signal representative of power reflected from the generator output, and wherein the sensing circuitry includes a signal conditioning stage arranged to compensate for relatively slow changes in the reflected power signal to produce the monitoring signal as a signal which is predominantly a function of relatively rapid changes in the reflected power signal.

**6.** A generator according to claim 1, wherein the signal conditioning stage includes a dual-channel arrangement feeding a comparator, one of the channels including an averaging element having a time-constant which is longer than that associated with the other channel, each channel feeding a respective input of the comparator, the monitoring signal being obtained at the output of the comparator.

**7.** A generator according to claim 1 or claim 2, wherein the sensing circuitry includes a demodulator for demodulating modulation of a reflected power signal.

**8.** A generator according to claim 6, wherein the demodulator is an amplitude demodulator.

**9.** A generator according to claim 6 or claim 7, wherein the sensing circuitry includes:

a first part associated with an output line coupling the power source to the generator output, the first part being arranged to generate a reflected power signal which is a voltage or current signal representative of power reflected from the generator output; and
a second part coupled to the first part and taking the form of a detector for detecting modulation of the reflected power signal and arranged to produce a detection signal which is a function of a predetermined characteristic of the modulation such as amplitude, phase or frequency, and
a third part including a signal conditioning stage arranged to compensate for relatively slow changes in the said modulation characteristic to produce the monitoring signal as a signal which is predominantly a function of relatively rapid changes in the said modulation characteristic.

**10.** A generator according to claim 8, wherein the signal conditioning stage includes a dual-channel arrangement feeding a comparator, one of the channels including an averaging element having a time-constant which is longer than that associated with the other channel, each channel feeding a respective input of the comparator, the monitoring signal being obtained at the output of the comparator.

**11.** A generator according to any of claims 6 to 9, wherein the sensing circuitry and the control arrangement are adapted to cause a reduction in the output power of the power source when the modulation, as represented by the detection signal, has a modulation characteristic exceeding a predetermined threshold level.

**12.** A generator according to any of claims 6 to 9, wherein the sensing circuitry and the control arrangement are adapted to cause a reduction in the output power of the power source when the modulation, as represented by the detection signal, substantially ceases after a period in which the said modulation characteristic exceeds a predetermined threshold level.

**13.** A generator according to claim 10 or claim 11, wherein the said modulation characteristic is amplitude modulation.

**14.** A generator according to any preceding claim, wherein the power source has an upper frequency part operable above 300 MHz and a lower frequency part operable below 300 MHz, both parts being coupled to the generator output, and wherein the sensing circuitry includes a load impedance sensor for sensing load impedance at a frequency or frequencies of operation of the lower frequency part of the source, the control arrangement being adapted to reduce or stop the application of radio frequency from the upper frequency part of the source in response to a predetermined load impedance condition sensed at the said operation frequency or frequencies of the lower frequency part of the source.

**15.** A generator according to claim 14, wherein the load impedance sensor comprises output voltage and current measuring circuitry coupled to an output line between the said lower frequency part of the source and the generator output.

**16.** A generator according to claim 14, wherein the frequency of operation of the lower frequency part of the source is dependent on load impedance and the load impedance sensor senses the frequency of the said lower frequency part.

**17.** A generator according to any one of claims 14 to 16, wherein the control arrangement is adapted to reduce or stop the application of power from the upper frequency part of the source when the sensed load impedance exceeds a predetermined threshold.

**18.** A generator according to any one of claims 14 to 17, wherein the lower frequency part of the source and the impedance sensor are arranged such that impedance sensing may be performed with application to the generator output of a signal which is of a low enough level to have a negligible electrosurgical effect, the frequency or frequencies of operation of the lower frequency source part being in the range of from 100kHz to 40MHz.

**19.** An electrosurgical generator comprising a radio frequency (r.f.) power source having an upper frequency part operable above 300 MHz and a lower frequency part operable below 300 MHz, a generator output for connection to an electrosurgical electrode assembly, both parts of the power source being coupled to the output, sensing circuitry including a load impedance sensor for sensing load impedance at a frequency or frequencies of operation of the lower frequency part of the source, and a control arrangement having an input coupled to the sensing circuitry to process a monitoring signal from the sensing circuitry, and a power control output coupled to the power source for adjusting the power output of the source, wherein the control arrangement is adapted to reduce or stop the application of r.f. power from the upper frequency part of the source to the generator output in response to a predetermined condition of the monitoring signal indicative of a change in load impedance sensed at the said operation frequency or frequencies of the lower frequency part of the source.

**20.** A generator according to claim 19, wherein the load impedance sensor comprises output voltage and current measuring circuitry coupled to an output line between the said lower frequency part of the source and the generator output.

**21.** A generator according to claim 19, wherein the frequency of operation of the lower frequency part of the source is dependent on load impedance and the load impedance sensor senses the frequency of the said lower frequency part.

**22.** A generator according to any one of claims 19 to 21, wherein the control arrangement is adapted to reduce or stop the application of power from the upper frequency part of the source when the sensed load impedance exceeds a predetermined threshold.

**23.** A generator according to any of claims 19 to 22, wherein the lower frequency part of the source and the impedance sensor are arranged such that impedance sensing may be performed with application to the generator output of a signal which is of a low enough level to have a negligible electrosurgical effect, the frequency or frequencies of operation of the lower frequency source part being in the range of from 100kHz to 40MHz.

**24.** An electrosurgical system comprising an electrosurgical generator according to any preceding claim, and coupled to the generator output, an electrode assembly including a tissue treatment electrode.

FIG. 1A

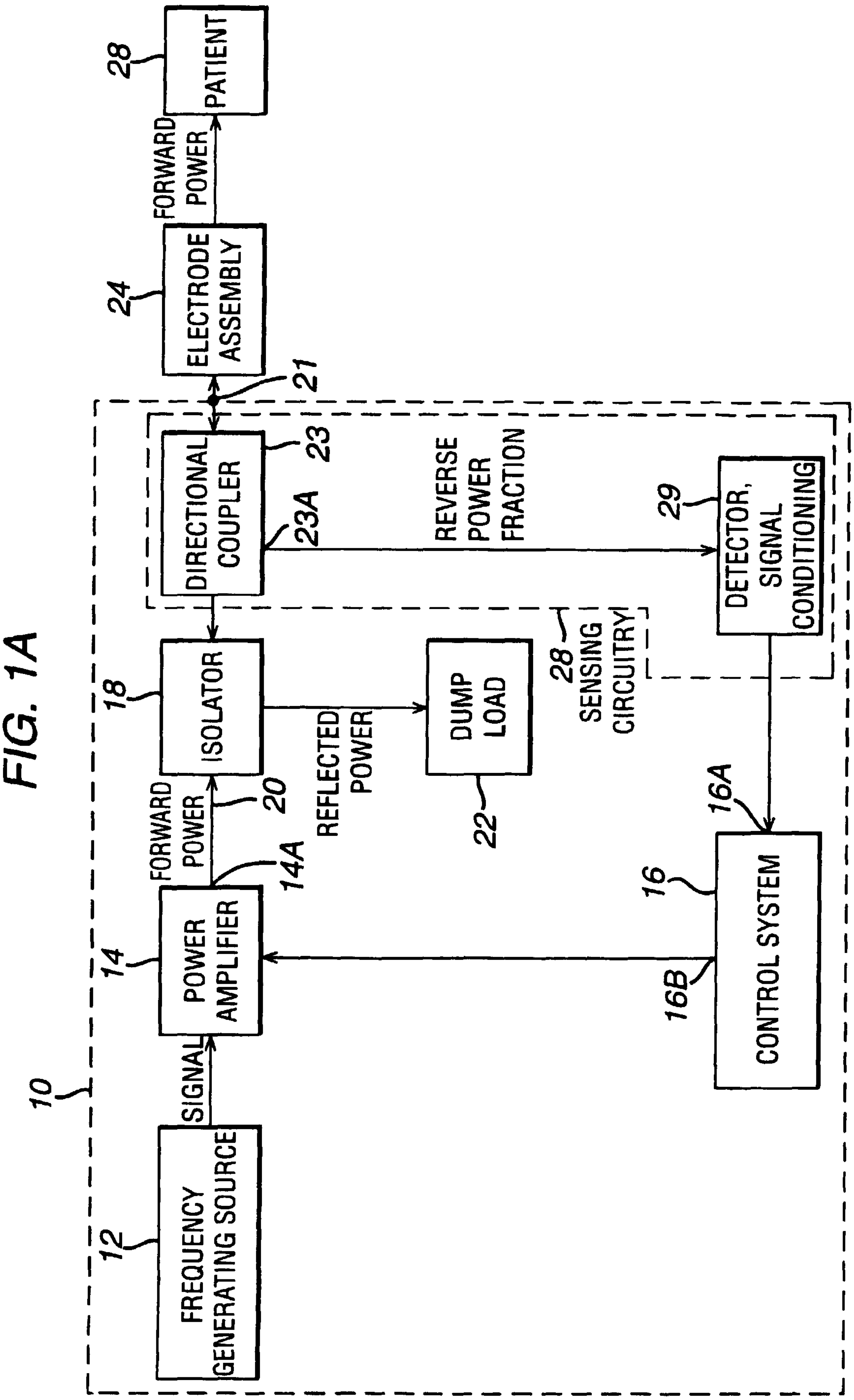
FREQUENCY GENERATING SOURCE
SIGNAL
POWER AMPLIFIER
FORWARD POWER
ISOLATOR
REFLECTED POWER
DUMP LOAD
DIRECTIONAL COUPLER
REVERSE POWER FRACTION
DETECTOR, SIGNAL CONDITIONING
SENSING CIRCUITRY
CONTROL SYSTEM
ELECTRODE ASSEMBLY
FORWARD POWER
PATIENT
10
12
14
14A
16
16A
16B
18
20
21
22
23
23A
24
28
28
29

FIG. 1B

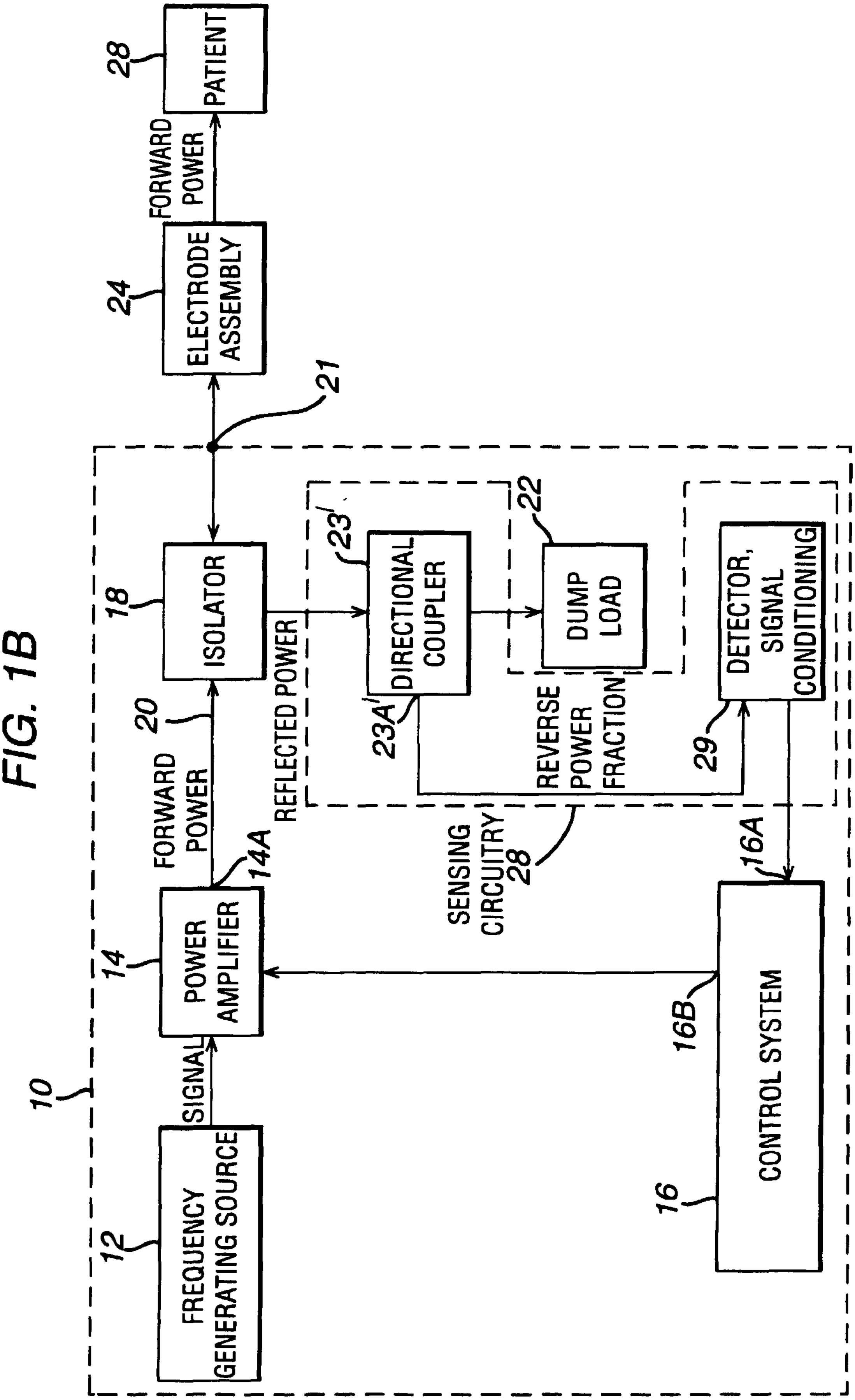
10
12
FREQUENCY GENERATING SOURCE
SIGNAL
14
POWER AMPLIFIER
14A
FORWARD POWER
20
18
ISOLATOR
REFLECTED POWER
21
24
ELECTRODE ASSEMBLY
FORWARD POWER
28
PATIENT
23'
DIRECTIONAL COUPLER
23A'
22
DUMP LOAD
REVERSE POWER FRACTION
DETECTOR, SIGNAL CONDITIONING
29
SENSING CIRCUITRY
28
16A
16B
16
CONTROL SYSTEM

FIG. 1C

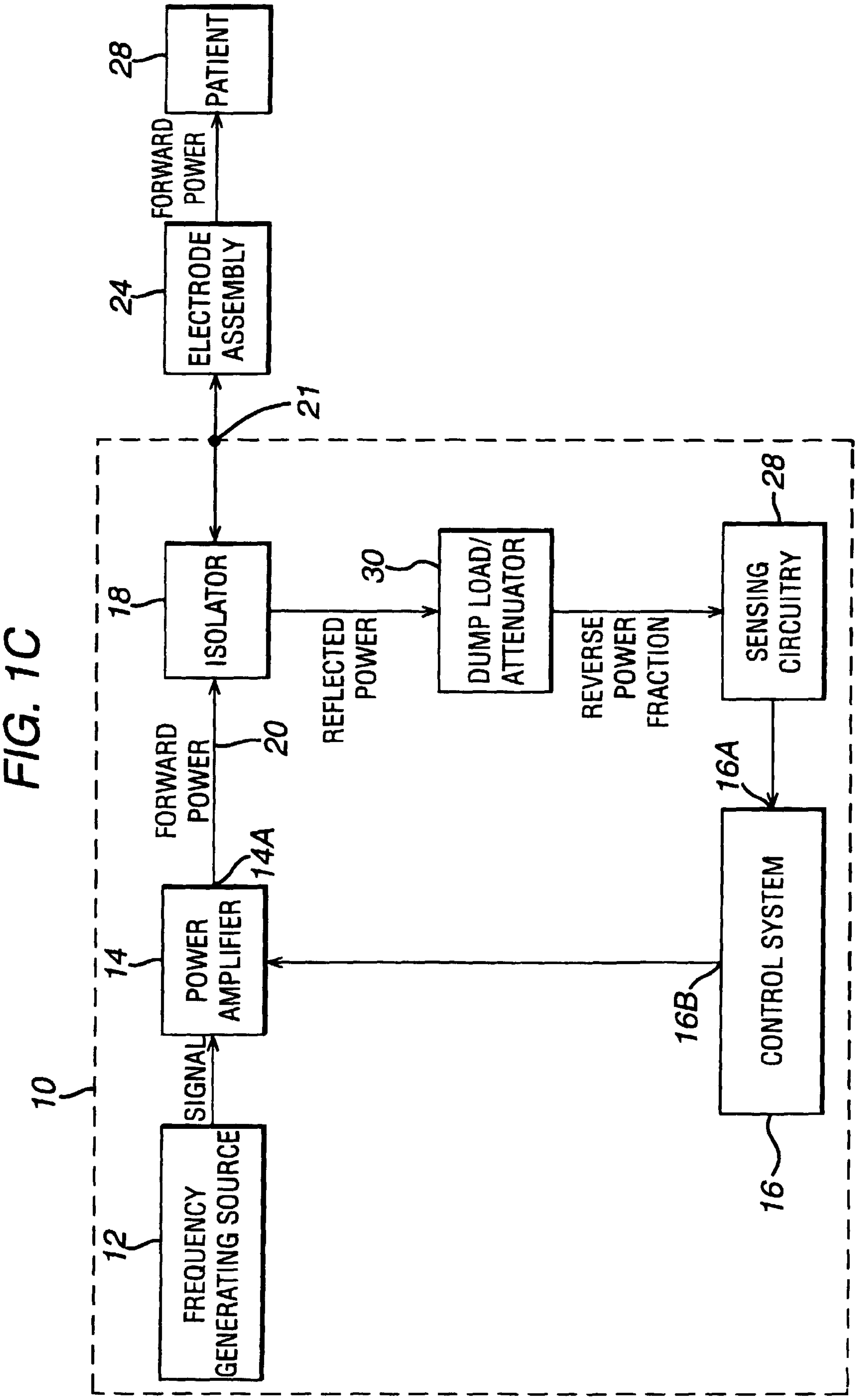
10
12
FREQUENCY GENERATING SOURCE
SIGNAL
14
POWER AMPLIFIER
14A
FORWARD POWER
20
18
ISOLATOR
21
24
ELECTRODE ASSEMBLY
FORWARD POWER
28
PATIENT
REFLECTED POWER
30
DUMP LOAD/ ATTENUATOR
REVERSE POWER FRACTION
28
SENSING CIRCUITRY
16A
CONTROL SYSTEM
16B
16

FIG. 2A

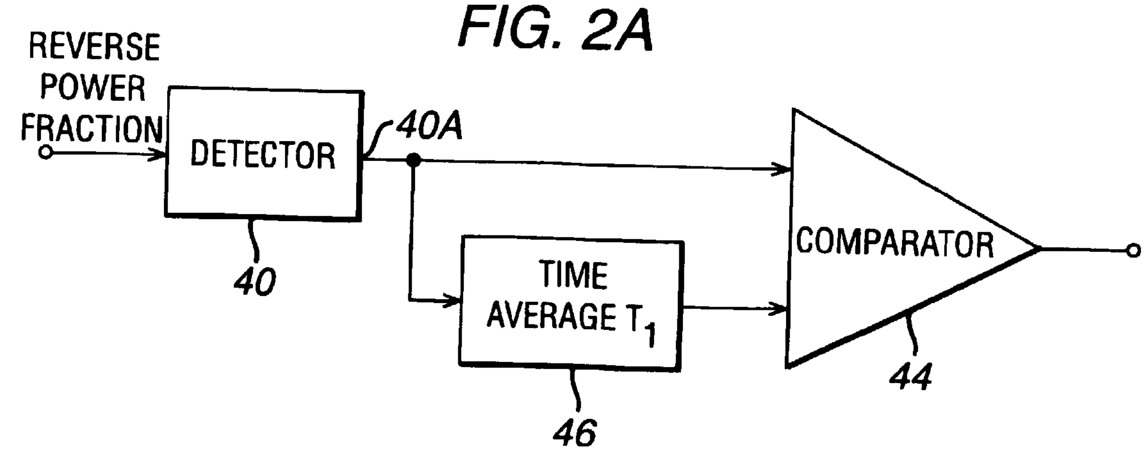


FIG. 2B

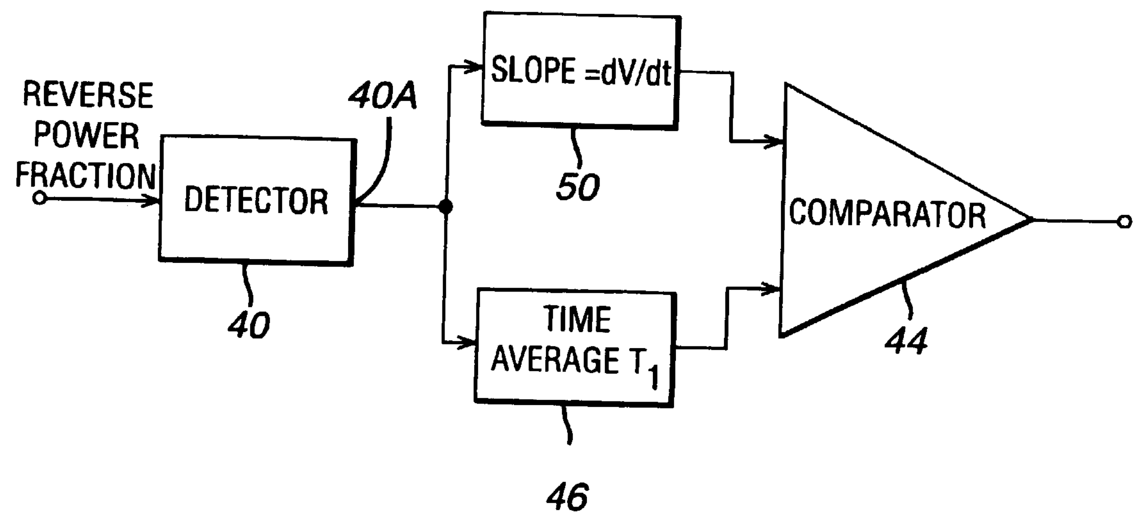


FIG. 2C

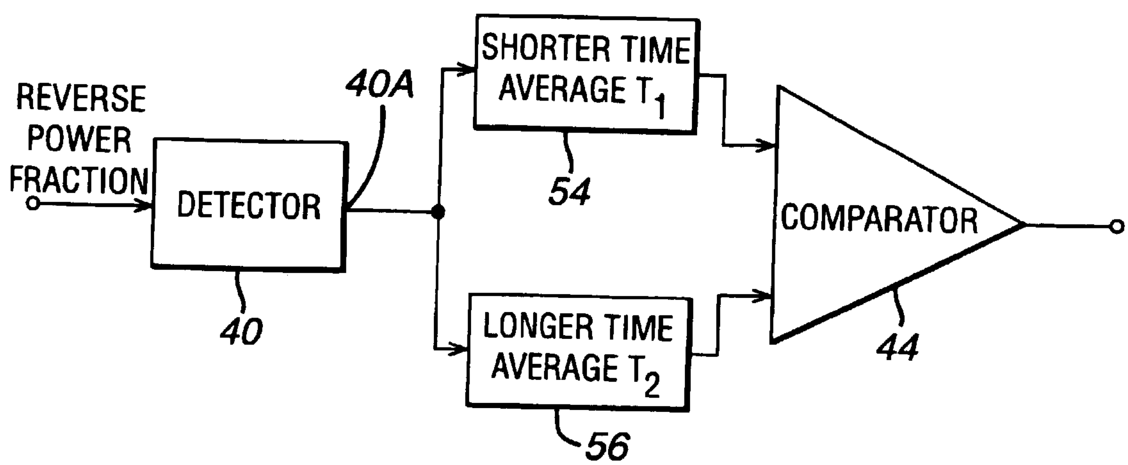

FIG. 3

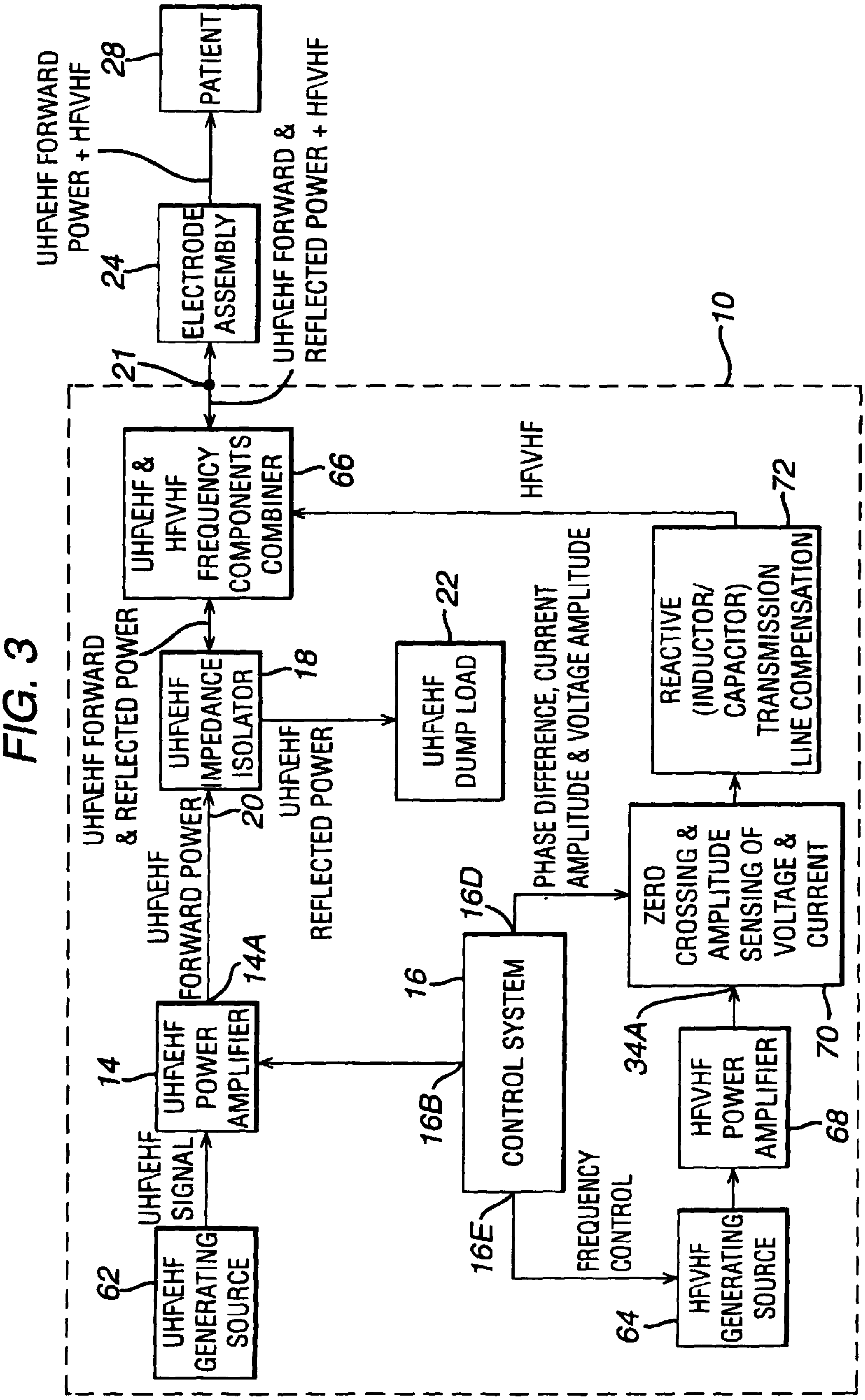
UHF\EHF FORWARD POWER + HF\VHF
28
PATIENT
24
ELECTRODE ASSEMBLY
UHF\EHF FORWARD & REFLECTED POWER + HF\VHF
21
10
UHF\EHF & HF\VHF FREQUENCY COMPONENTS COMBINER
66
HF\VHF
72
REACTIVE (INDUCTOR/ CAPACITOR) TRANSMISSION LINE COMPENSATION
UHF\EHF FORWARD & REFLECTED POWER
UHF\EHF IMPEDANCE ISOLATOR
18
20
22
UHF\EHF DUMP LOAD
UHF\EHF REFLECTED POWER
PHASE DIFFERENCE, CURRENT AMPLITUDE & VOLTAGE AMPLITUDE
UHF\EHF FORWARD POWER
14A
16D
16
CONTROL SYSTEM
16B
ZERO CROSSING & AMPLITUDE SENSING OF VOLTAGE & CURRENT
70
34A
14
UHF\EHF POWER AMPLIFIER
HF\VHF POWER AMPLIFIER
68
UHF\EHF SIGNAL
62
UHF\EHF GENERATING SOURCE
16E
FREQUENCY CONTROL
64
HF\VHF GENERATING SOURCE

FIG. 4

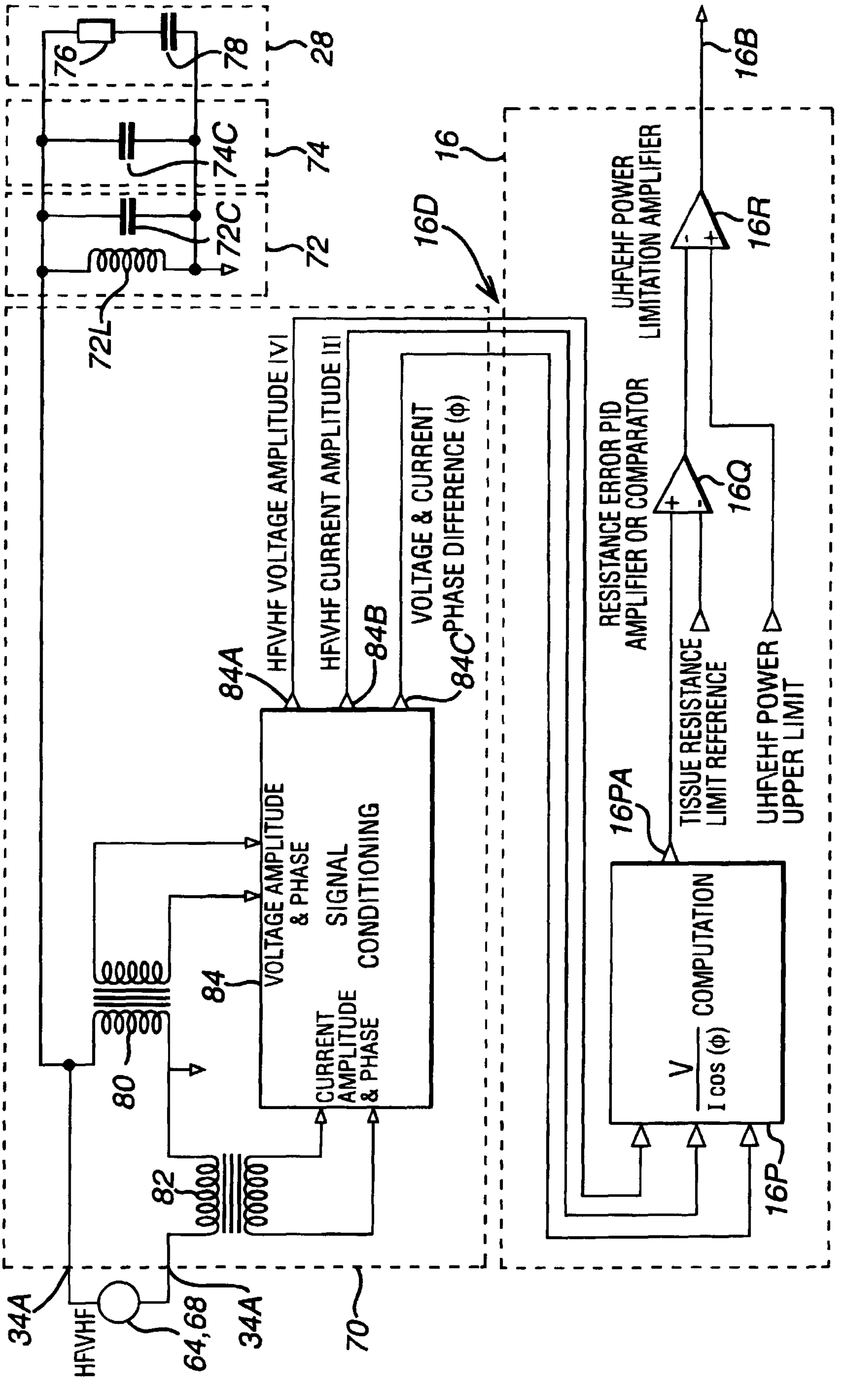

HF\VHF
34A
64,68
34A
70
80
82
84
72L
72
72C
74
74C
76
78
28
16D
16
16B
16R
16Q
16PA
16P
84A
84B
84C
VOLTAGE AMPLITUDE & PHASE
SIGNAL CONDITIONING
CURRENT AMPLITUDE & PHASE
HF\VHF VOLTAGE AMPLITUDE |V|
HF\VHF CURRENT AMPLITUDE |I|
VOLTAGE & CURRENT PHASE DIFFERENCE (ϕ)
V / I cos (ϕ) COMPUTATION
TISSUE RESISTANCE LIMIT REFERENCE
UHF\EHF POWER UPPER LIMIT
RESISTANCE ERROR PID AMPLIFIER OR COMPARATOR
UHF\EHF POWER LIMITATION AMPLIFIER

European Patent Office

# EUROPEAN SEARCH REPORT

Application Number
EP 00 30 4252

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br>A | EP 0 752 235 A (TARGET THERAPEUTICS INC)<br>8 January 1997 (1997-01-08)<br>* column 5, line 8-21; claims 1-5 * | 1<br>19 | A61B18/12 |
| A | US 5 904 709 A (ARNDT G DICKEY ET AL)<br>18 May 1999 (1999-05-18)<br>* column 10, line 19-49 * | 19 | |
| A | US 5 628 745 A (BEK ROBIN B)<br>13 May 1997 (1997-05-13)<br>* abstract * | 1,19 | |
| P,A | US 5 954 686 A (ELLMAN ALAN G ET AL)<br>21 September 1999 (1999-09-21) | | |

TECHNICAL FIELDS SEARCHED (Int.Cl.7)
A61B

The present search report has been drawn up for all claims

| Place of search | Date of completion of the search | Examiner |
|---|---|---|
| THE HAGUE | 14 September 2000 | Papone, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 30 4252

This annex lists the patent family members relating to the patent documents cited in the above-mentioned European search report.
The members are as contained in the European Patent Office EDP file on
The European Patent Office is in no way liable for these particulars which are merely given for the purpose of information.

14-09-2000

| Patent document cited in search report | Publication date | Patent family member(s) | Publication date |
|---|---|---|---|
| EP 0752235 A | 08-01-1997 | US 6019757 A | 01-02-2000 |
| | | AU 681060 B | 14-08-1997 |
| | | AU 5940496 A | 30-01-1997 |
| | | CA 2180750 A | 08-01-1997 |
| | | JP 2957949 B | 06-10-1999 |
| | | JP 9149904 A | 10-06-1997 |
| | | NO 962840 A | 08-01-1997 |
| US 5904709 A | 18-05-1999 | US 6047216 A | 04-04-2000 |
| US 5628745 A | 13-05-1997 | AT 192310 T | 15-05-2000 |
| | | AU 714068 B | 16-12-1999 |
| | | AU 5700896 A | 24-12-1996 |
| | | CA 2221285 A | 12-12-1996 |
| | | DE 69608104 D | 08-06-2000 |
| | | EP 0833592 A | 08-04-1998 |
| | | WO 9639087 A | 12-12-1996 |
| | | JP 2960971 B | 12-10-1999 |
| | | JP 10507394 T | 21-07-1998 |
| US 5954686 A | 21-09-1999 | NONE | |

EPO FORM P0459

For more details about this annex : see Official Journal of the European Patent Office, No. 12/82